# EUROPEAN PATENT SPECIFICATION

(11) **EP 0 595 308 B1**
(45) Date of publication and mention of the grant of the patent: **12.12.2001**
(21) Application number: 93117442.9
(22) Date of filing: 27.10.1993
(51) Int. Cl.: A61M 25/00, A61M 25/10, A61M 29/02

(54) **Rapid exchange catheter**
Katheter, welcher schnell ausgetauscht werden kann
Cathéter à échange rapide

(30) Priority: 30.10.1992 US 969887; 30.10.1992 US 969946
(43) Date of publication of application: 04.05.1994
(73) Proprietor: Cordis Corporation, Miami Lakes Florida 33014 (US)
(72) Inventor: Solar, Ronald J., San Diego California 92131 (US)
(74) Representative: Ruschke, Hans Edvard, Dipl.-Ing.

(56) References cited:
- EP-A- 0 274 129
- EP-A- 0 365 993
- EP-A- 0 436 501
- EP-A- 0 476 807
- US-A- 4 944 745
- US-A- 5 059 177

## Description

### FIELD OF THE INVENTION

This invention is directed to an angioplasty apparatus for facilitating rapid exchanges. More particularly, this invention is directed to a rapid exchange catheter system whereby a double-lumen dilatation balloon catheter has an opening in one lumen adjacent its distal end for a guidewire and a pushing wire extending through the proximal portion of that same lumen.

### BACKGROUND OF THE INVENTION

During angioplasty procedures it is often necessary to exchange one dilatation catheter for another. To do so requires manipulation of lengthy exchange wires, which is time consuming and awkward to the extent that two operators are required. A current approach to dealing with this is the "monorail" system wherein a dilatation catheter has a structure such that only the distal portion of the catheter tracks a guidewire. Examples of such systems are described in Yock, U.S. Patents Nos. 5,040,548 and 5,061,273, Bonzel, U.S. Patent No. 4,762,129, and Kramer, U.S. Patent No. 5,135,535, all of which are incorporated herein by reference.

A further example of this type of prior art arrangement is shown in document EP-A-0 274 129, on which the pre-characterizing part of claim 1 is based.

In the known monorail systems the pushing force on the dilatation catheter is eccentric to the guidewire, such that there is not total responsiveness in the system as the operator attempts to manipulate the dilatation catheter along the guidewire. This can cause binding and failure to move the catheter through tortuous arterial segments and tight stenoses. Furthermore, in these systems/designs, the guidewire lumen is positioned coaxially within a balloon that is attached to the catheter shaft at the proximal and distal ends of the balloon. This arrangement allows the balloon to compress along the guidewire lumen, increasing in profile, and thereby also causing binding and failure to move the catheter. Dependent upon the clinical application, balloons of varying lengths may be required. In addition to the time and expense required to develop and qualify separate balloons for each application, as the balloon length increases the tendency for binding increases. Thus, there is a need for a monorail type system wherein there will be co-linear design between a push wire and the guidewire and a parallel arrangement between the balloon and the guidewire lumen, resulting in more positive tracking and facilitated passage through tortuous arterial segments and tight stenoses, as well as simplified methods of manufacture.

### OBJECTS OF THE INVENTION

It is an object of the invention to provide a balloon dilatation system capable of rapid exchange.

It is also an object of the invention to provide a balloon dilatation system wherein a double lumen dilatation catheter has a pushing wire that extends through one lumen to a position adjacent or proximal to the dilatation balloon.

It is a further object of the invention to provide a double lumen dilatation catheter wherein the guidewire lumen is positioned exterior to the dilatation balloon.

It is yet a further object of the invention to provide a rapid exchange balloon dilatation catheter that is simple to manufacture.

These and other objects of the invention will become more apparent from the description below.

### BRIEF DESCRIPTION OF THE DRAWINGS

Fig. 1 depicts a cross-sectional view of the distal portion of a balloon dilatation catheter according to the invention;
Fig. 2 represents a cross-sectional view along the line 2-2;
Figs. 3, 5, and 7 represent perspective views of a distal portion of the catheter of the invention as it is being formed; and
Figs. 4, 6, and 8 represent cross-sectional views of work pieces from which the catheter of the invention can be formed.

### DETAILED DESCRIPTION OF THE INVENTION

According to the invention there is provided a balloon dilatation catheter which comprises: an elongate flexible tubular shaft having a proximal end and a distal end; an inflatable dilatation balloon forming part of the distal end of the shaft, the dilatation balloon having a proximal end and a distal end; a first, inflation lumen extending through the shaft and having a proximal end and a distal end, the distal end of the first lumen opening into and being in fluid communication with the dilatation balloon; a second lumen extending through the shaft and having a proximal end and a distal end, the distal end of the second lumen being open and the second lumen having a distal portion defined by (i) an opening) proximal to the distal end of the second lumen and (ii) the distal end of the second lumen, and a proximal portion) defined by the portion of the second lumen proximal to said opening; a guidewire received in the distal portion of the second lumen; and a pushing wire having a proximal end and a distal end and extending through the second lumen, the distal end of the pushing wire being fixed in the second lumen proximal to the distal end of the second lumen ; characterised in that said distal portion of the second lumen is enlarged, compared with the proximal portion, to facilitate receiving the guidewire in a sliding fit and the guidewire has a slitter for slitting the enlarged distal portion as the catheter is withdrawn to separate the catheter from the guidewire.

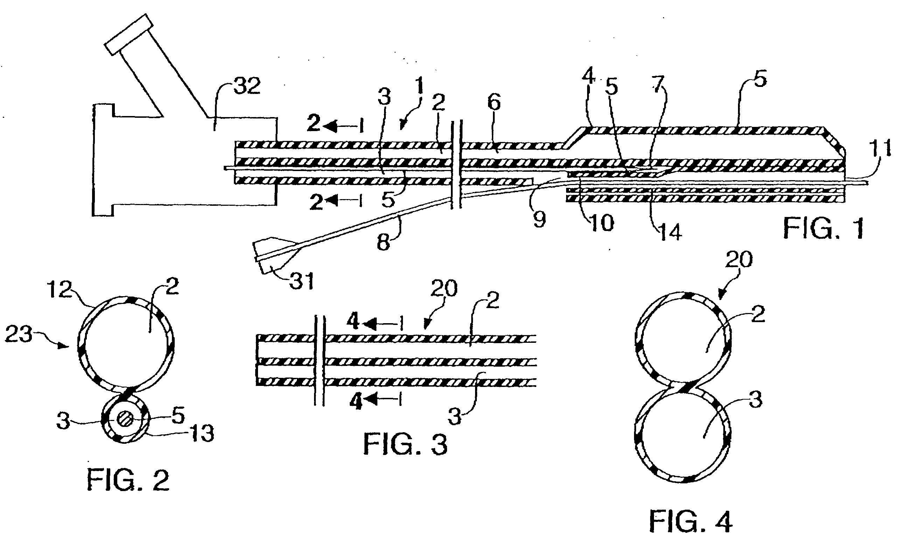

The invention can perhaps be better appreciated by making reference to the drawings. Figure 1 depicts the distal portion of a balloon dilatation catheter 1 having coextensively extending lumens 2 and 3. Lumen 2 terminates in a dilatation balloon 4 which is inflated and deflated through lumen 2. Lumens 2 and 3 are preferably each in fluid communication with a hub or fluid communication means 32.

Lumen 3 contains pushing wire 5, which extends from the proximal end (not shown) of catheter 1 to a position 6 proximal or adjacent to balloon 4. The distal portion of pushing wire 5 is secured at position 6 by closure, e.g., heat-shrinking, of lumen 3 or by insertion of a plug or other holding means. Also, the distal portion 7 of pushing wire 5 is preferably tapered distally to provide a smooth transition in axial stiffness. The pushing wire 5 will become less stiff as the diameter of pushing wire 5 narrows in the distal direction. The tapering is substantially linear over the distal portion of the pushing wire 5. Such tapering can extend from about 2 to 40 cm from the distal end of the pushing wire 5. Optionally, instead of linear tapering, the tapering may be stepped, in discrete reductions, or otherwise nonlinear.

The distal portion of a guidewire 8 is threaded through opening 9 into the enlarged section, i.e., guidewire lumen, 10 of lumen 2. As the guidewire 8 is threaded into section 10, it exits through distal opening 11.

Figure 2 represents a cross-sectional view showing how lumens 2 and 3 relate to one another and how pushing wire 5 is positioned within lumen 3. Lumen walls 12 and 13 can each have a thickness of from about 7.62 µm to 508 µm (0.3 to 20 mil), preferably from about (12.7 µm to 254 µm) 0.5 to 10 mil. Lumen wall 13 will most likely be slightly thicker than lumen wall 12.

The lumen walls 12 and 13 are comprised of materials conventional to balloon dilatation catheters. Suitable materials include polyolefins such as polyethylene, polyethylene terepthalate, polyurethanes, polyesters, and various copolymers thereof. Pushing wire 5 can be made from any rigid, medically acceptable material suitable for such use, including, but not limited to wires or hypotubes comprised of stainless steel or other rigid materials.

The construction according to the invention leads to flexibility in product design. For example, the choice of pushing wire allows the designer to impart various features to the catheter in the form of various flexibility and pushability combinations. Also, a hollow pushing wire, or deletion or removal of the pushing wire, would facilitate infusion of fluids, drugs, and/or contrast media through the catheter into the distal vasculature. Further, it is within the scope of the invention that catheter 1 may have at least one additional, coextensive lumen that would similarly facilitate infusion of liquids, drugs and/or contrast media. For example, a catheter 1 with a third, coextensive lumen open at its distal end could have several possible applications.

In a preferred embodiment of the invention, as shown in Fig. 1, a lubricious coating or a section of thin tubing 14 of lubricious material is sealed into enlarged section 10. There are several known materials suitable for this purpose, such as polytetrafluoroethylene (available as TEFLON® from duPont), polyethylenes, polysiloxanes, etc. In this embodiment the tubing section 14 can hold the distal portion 7 of pushing wire 5 in position.

As shown in Figure 1 a slitting means 31 is mounted proximally on guidewire 8. Then, as the catheter is withdrawn, the enlarged section 10 engages the slitter, the enlarged section 10 is slit, and catheter 1 is separated from guidewire 8. This would eliminate the requirement for the operator to change hands as catheter 1 is removed.

The catheter 1 may have visual length markings along its shaft that would enable the operator to predict when the catheter 1 would exit the guiding catheter into the vasculature. This would reduce the fluoroscopy time. The preferred design would put the markings directly on the pushing wire 5 (heat shrink tubing rings, inks, paints, etc.). Since the pushing wire 5 is encapsulated within the second lumen 3, the markings would not be exposed to the patient (i.e., markings would not come off, and materials which could be toxic if exposed may be used).

Example of preparation of a catheter 1 according to the invention is shown in Figures 3 to 8. After a double lumen workpiece 20 is prepared, the distal end of the workpiece is sealingly clamped, and heat and inflation pressure are applied to cause the distal portion of lumen 2 to expand to form the wall of balloon 4 and the distal portion of lumen 3 to expand to form enlarged section 10. The location where heat is applied can be varied to vary the respective lengths of balloon 4 and enlarged section 10. Heat sealing or application of suitable adhesive seals the distal portion of balloon 4. Opening 9 is cut into section 10, and opening 11 is maintained or created by trimming the distal portion of the catheter 1. Pushing wire 5 is then inserted into lumen 3, wherein either the remaining proximal portion 18, or more, of lumen 3 is heat shrunk to cause pushing wire 5 to be positively engaged by lumen 3. Alternatively, the distal portion of pushing wire 5 could be affixed by suitable means, such as an adhesive or a plug, in lumen 3.

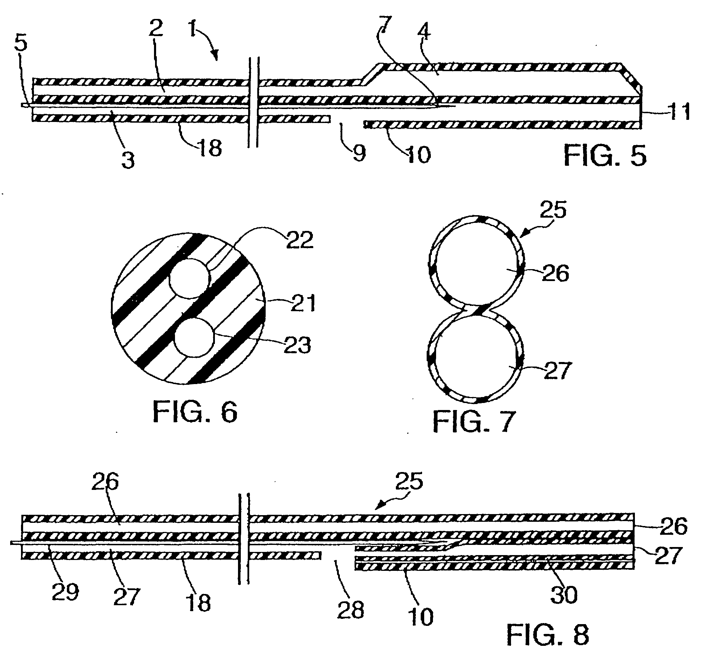

Workpiece 20 can be prepared by methods well-known to those skilled in the art. In a preferred method workpiece 20 can be prepared by blowing extruded tubing 21, a cross-section of which is shown in Fig. 6.

Catheter 1 can be prepared from extruded tubing 21 by blowing said tubing 21 under pressure and heating conditions sufficient to produce a catheter piece 25, a cross-section of which is shown in Fig. 7, where the diameters of lumens 26 and 27 correspond substantially to the final diameters of balloon 4 and enlarged section 10, respectively. The holes or openings 22 and 23 in tubing 21 are not necessarily the same, such that the diameters of lumens 26 and 27 may also differ.

After an opening 28 (corresponding to opening 9) is cut into lumen 27 at a point to define the length of the guidewire lumen or enlarged section 10, a pushing wire 29 is inserted into lumen 27. Pushing wire 29 extends the length of lumen 27 to a point slightly distal of opening 28. Preferably a lubricious liner 30 is inserted into the distal end of lumen 27. Then, the distal end of lumen 26 is sealed, and, while lumen 26 is pressurized, heat is applied to the distal portion of catheter piece 25 to cause lumen 27 to slightly shrink around liner 30, which fixedly engages the distal end of pushing wire 29. Next, the portion of catheter piece 25 proximal to the balloon is heated to shrink lumen 26 and to shrink lumen 27 around pushing wire 29. The balloon length is determined by the location where heat is applied to lumen 26.

When portions of the catheter are heated, the heating can be effected by a point source of heat, where the point source is moved along the exterior of the catheter or the catheter is moved across the point source. Alternatively, the heat can be applied with a broader heat source, such as a hot water bath. The source of and/or techniques of heating will be apparent to those skilled in the art.

The workpiece will optionally be cross-linked prior to working. Such cross-linking could be effected by chemical or irradiation means. The workpiece can be optionally or additionally oriented by mechanical means, such as stretching during blowing.

The catheters of the invention are prepared by use of techniques and procedures known to those skilled in the art. For example, the pressure and heating conditions will vary according to the materials used and the results desired, and it is well within the skill of those skilled in the art to determine the proper pressure and heating requirements.

An additional advantage of the design and preparation described is that the catheter can be of integral design and multiple bonding steps can be avoided. The balloon and both lumens can be formed from a single piece. This design permits improvements in manufacturing yields, quality, and reliability due to simplified construction.

Guidewire 8 may be a conventional guidewire, preferably a spring guidewire, as is well known. Typical guidewires are shown in U.S. Patents Nos. 4,757,827, 4,815,478, 4,813,434, 4,619,274, 4,554,929, 4,545,390, 4,538,622, 3,906,938, 3,973,556, and 4,719,924. In addition, the shaft of guidewire 8 could be solid or hollow, such as a hypotube, with an open distal end, to facilitate drug infusion.

Operation and use of the angioplasty apparatus shown in Fig. 1 may now be briefly described as follows: A guiding catheter is inserted into the coronary artery in a conventional manner. The guidewire 8 is then introduced into the guiding catheter and advanced to and across the lesion. Now, the balloon dilatation catheter is inserted onto the guidewire by a back loading technique, where the proximal extremity of the guidewire 8 is inserted backwardly through the tip 11 of the balloon dilatation catheter 1 through the enlarged section 10, and exits opening 9. The catheter 1 is then advanced along the guidewire 8 to and across the lesion.

After the balloon 4 has crossed the stenosis or lesion, the balloon 4 can be inflated in a conventional manner by introducing a radiopaque contrast liquid through the lumen 2. After the inflation has occurred and the desired operation has been performed by enlarging the opening in the stenosis, the balloon dilatation catheter 1 can be removed very rapidly by holding the guidewire 8 stationary and withdrawing the balloon dilatation catheter.

If it is ascertained by the operator that additional dilatation of the stenosis is desired and that a larger balloon should be inserted into the stenosis, this can be accomplished very rapidly by selecting the desired size of balloon dilation catheter and repeating the aforementioned procedure. The balloon of the new dilatation catheter can be inflated in the same manner as hereinbefore described. If necessary, even another exchange procedure can be readily accomplished in the same manner as hereinbefore described utilizing a still larger balloon dilatation catheter if that turns out to be necessary.

After the desired amount of dilation of the stenosis or lesion has been accomplished, the balloon dilatation catheter can be removed and thereafter the guiding catheter can be removed.

The preceding specific embodiments are illustrative of the practice of the invention. It is to be understood, however, that other expedients known to those skilled in the art or disclosed herein, may be employed without departing from the scope of the appended claims.

## Claims

1. A balloon dilatation catheter which comprises:
an elongate flexible tubular shaft (1) having a proximal end and a distal end;
an inflatable dilatation balloon (4) forming part of the distal end of the shaft, the dilatation balloon (4) having a proximal end and a distal end;
a first, inflation lumen (2) extending through the shaft (1) and having a proximal end and a distal end, the distal end of the first lumen opening into and being in fluid communication with the dilatation balloon (4);
a second lumen (3) extending through the shaft and having a proximal end and a distal end, the distal end of the second lumen (3) being open and the second lumen (3) having a distal portion (10) defined by (i) an opening (9) proximal to the distal end of the second lumen (3) and (ii) the distal end of the second lumen (3), and a proximal portion (18) defined by the portion of the second lumen (3) proximal to said opening;
a guidewire (8) received in the distal portion (10) of the second lumen (3); and
a pushing wire (5) having a proximal end and a distal end and extending through the second lumen (3), the distal end of the pushing wire (5) being fixed in the second lumen (3) proximal to the distal end of the second lumen (3);
**characterised in that**
said distal portion (10) of the second lumen (3) is enlarged, compared with the proximal portion (18), to facilitate receiving the guidewire (8) in a sliding fit and the guidewire (8) has a slitter for slitting the enlarged distal portion (10) as the catheter is withdrawn to separate the catheter from the guidewire.

2. The catheter of Claim 1, wherein the opening (9) of the second lumen (3) is proximal or adjacent to the distal end of the dilatation balloon (4).

3. The catheter of Claim 1, wherein the distal end (7) of the pushing wire (5) is positioned at or near the opening (9) of the second lumen (3).

4. The catheter of Claim 1, wherein the enlarged portion (10) of the second lumen (3) comprises lubricious material.

5. The catheter of Claim 4, wherein the lubricious material is in the form of tubing (14) within the enlarged distal portion (10) of the second lumen (3).

6. The catheter of Claim 5, wherein the enlarged distal portion (10) has an inner surface and the distal end (7) of the pushing wire (5) is held in position by the lubricious tubing (14) and said inner surface.

7. The catheter of Claim 1, wherein the proximal portion (18) of the second lumen (3) proximal to the opening (9) has been heat shrunk around the pushing wire (5).

8. The catheter of Claim 1, wherein the proximal end of the first lumen (2) is connected to a fluid communication means (32) such that the first lumen (2) is in fluid communication with an inflation source.

9. The catheter of Claim 1 which comprises at least one additional lumen extending through the tubular shaft (1) and having open proximal and distal ends.

10. The catheter of Claim 9, wherein there is one additional lumen.

11. The catheter of Claim 10, wherein the proximal end of the additional lumen is connected to a fluid communication means (32) such that the distal end of the lumen is in fluid communication with the fluid communication means (32).

12. A balloon dilatation catheter system which comprises a guidewire (8) and two or more catheters (1) of Claim 1, which catheters (1) can be sequentially exchanged over said guidewire (8).

13. The system of Claim 12, wherein each catheter (1) has a dilatation balloon (4) of a different size.

## Patentansprüche

1. Ballondilatationskatheter mit
einem langgestreckten flexiblen rohrförmigen Schaft (1) mit einem proximalen und einem distalen Ende,
einem aufweitbaren Dilatationsballon (4), der Teil des distalen Schaftendes ist und ein proximales und ein distales Ende hat,
einem ersten Aufweitlumen (2), das durch den Schaft (1) verläuft und ein proximales und ein distales Ende hat, wobei das distale Ende des ersten Lumens in den Dilatationsballon (4) mündet und in Strömungsverbindung mit diesem steht,
einem zweiten Lumen (3), das durch den Schaft verläuft und ein proximales und ein distales Ende hat, wobei das zweite Lumen (3) offen ist und einen distalen Bereich (10), der von (i) einer zum distalen Ende des zweiten Lumens (3) distalen Öffnung (9) und von (ii) dem distalen Ende des zweiten Lumens (3) gebildet wird, sowie einen proximalen Bereich (18) aufweist, der von dem zur Öffnung proximalen Bereich des zweiten Lumens gebildet wird,
einem Führungsdraht (8), der vom distalen Bereich (10) des zweiten Lumens (3) aufgenommen wird, und
einem Schiebedraht (5) mit einem proximalen und einem distalen Ende, der durch das zweite Lumen (3) verläuft, wobei das distale Ende des Schiebedrahts (5) im zweiten Lumen (3) proximal zum distalen Ende des zweiten Lumens (3) festgelegt ist,
**dadurch gekennzeichnet, dass**
der distale Bereich (10) des zweiten Lumens (3) gegenüber dem proximalen Bereich (18) aufgeweitet ist, um die Aufnahme des Fühnrungsdrahts (8) in einer Gleitpassung zu erleichtern, und dass der Führungsdraht (8) eine Aufschlitzeinrichtung aufweist, um den aufgeweiteten distalen Bereich (10) beim Herausziehen des Katheters zum Trennen desselben von Führungsdraht aufzuschlitzen.

2. Katheter nach Anspruch 1, bei dem die Öffnung (9) des zweiten Lumens (3) proximal am distalen Ende des Dilatationsballons (4) liegt bzw. an dieses angrenzt.

3. Katheter nach Anspruch 1, bei dem das distale Ende (7) des Schiebedrahts (5) an bzw. nahe der Öffnung (9) des zweiten Lumens (3) positioniert ist.

4. Katheter nach Anspruch 1, bei dem der aufgeweitete Bereich (10) des zweiten Lumens (3) ein gleitförderndes Material aufweist.

5. Katheter nach Anspruch 4, bei dem das gleitfördernde Material als Schlauchstück (14) im aufgeweiteten Bereich (10) des zweiten Lumens (3) vorliegt.

6. Katheter nach Anspruch 5, bei dem der aufgeweitete Bereich (10) eine Innenfläche aufweist und das distale Ende (7) des Schiebedrahts (5) von dem gleitfördernden Schlauchmaterial (14) und der Innenfläche in der Solllage gehalten wird.

7. Katheter nach Anspruch 1, bei dem der proximale Bereich (18) des zweiten Lumens (3) proximal der Öffnung (9) auf den Schiebedraht (5) aufgeschrumpft worden ist.

8. Katheter nach Anspruch 1, bei dem das proximale Ende des ersten Lumens (2) mit einem Flüssigkeitsanschluss (32) verbunden ist derart, dass das erste Lumen (2) in Strömungsverbindung mit der Aufweitquelle steht.

9. Katheter nach Anspruch 1 mit mindestens einem zusätzlichen Lumen, das durch den rohrförmigen Schaft (1) verläuft und ein offenes proximales sowie ein distales Ende aufweist.

10. Katheter nach Anspruch 9, der ein zusätzliches Lumen aufweist.

11. Katheter nach Anspruch 10, bei dem das proximale Ende des zusätzlichen Lumens mit einem Flüssigkeitsanschluss (32) verbunden ist derart, dass das distale Ende des Lumens in Strömungsverbindung mit dem Flüssigkeitsanschluss (32) steht.

12. Ballondilatationskathetersystem mit einem Führungsdraht (8) und zwei oder mehr Kathetern (1) nach Anspruch 1, die im Austausch nacheinander auf den Führungsdraht (8) aufschiebbar sind.

13. System nach Anspruch 12, bei dem die Dilatationsballons (4) der Katheter (1) unterschiedlich groß sind.

## Revendications

1. Cathéter à ballon de dilatation comprenant :
◆ un arbre tubulaire souple allongé (1) ayant une extrémité proximale et une extrémité distale ;
◆ un ballon de dilatation gonflable (4) faisant partie de l'extrémité distale de l'arbre, le ballon de dilatation (4) ayant une extrémité proximale et une extrémité distale ;
◆ un premier lumen de gonflage (2) se prolongeant à travers l'arbre (1) et ayant une extrémité proximale et une extrémité distale, l'extrémité distale du premier lumen s'ouvrant dans et étant en communication fluide avec le ballon de dilatation (4) ;
◆ un deuxième lumen (3) se prolongeant à travers l'arbre et ayant une extrémité proximale et une extrémité distale, l'extrémité distale du deuxième lumen (3) étant ouverte et le deuxième lumen (3) comportant une partie distale (10) définie par (i) une ouverture (9) proximale vers l'extrémité distale du deuxième lumen (3) et (ii) l'extrémité distale du deuxième lumen (3), et une partie proximale (18) définie par la partie du deuxième lumen (3) proximale vers ladite ouverture ;
◆ un fil de guidage (8) reçu dans la partie distale (10) du deuxième lumen (3) ; et
◆ un fil de poussée (5) ayant une extrémité proximale et une extrémité distale et se prolongeant à travers le deuxième lumen (3), l'extrémité distale du fil de poussée (5) étant fixe dans le deuxième lumen (3) proximale vers l'extrémité distale du deuxième lumen (3) ;
**caractérisé en ce que** ladite partie distale (10) du deuxième lumen (3) est agrandie par rapport à la partie proximale (18), pour faciliter la réception du fil de guidage (8) dans une fente de coulissement et le fil de guidage (8) comporte un élément de fendage pour fendre la partie distale agrandie (10) lorsque le cathéter est extrait pour séparer le cathéter du fil de guidage.

2. Cathéter selon la revendication 1, dans lequel l'ouverture (9) du deuxième lumen (3) est proximale ou adjacente à l'extrémité distale du ballon de dilatation (4).

3. Cathéter selon la revendication 1, dans lequel l'extrémité distale (7) du fil de poussée (5) est positionnée sur ou près de l'ouverture (9) du deuxième lumen (3).

4. Cathéter selon la revendication 1, dans lequel la partie agrandie (10) du deuxième lumen (3) comprend un matériau de lubrification.

5. Cathéter selon la revendication 4, dans lequel le matériau de lubrification est sous la forme d'un tube (14) à l'intérieur de la partie distale agrandie (10) du deuxième lumen (3).

6. Cathéter selon la revendication 5, dans lequel la partie distale agrandie (10) possède une surface interne et l'extrémité distale (7) du fil de poussée (5) et maintenue en position par le tube de lubrification (14) et ladite surface interne.

7. Cathéter selon la revendication 1, dans lequel la partie proximale (18) du deuxième lumen (3) proximale vers l'ouverture (9) a été rétrécie à la chaleur autour du fil de poussée (5).

8. Cathéter selon la revendication 1, dans lequel l'extrémité proximale du premier lumen (2) est reliée à un moyen de communication fluide (32) de façon que le premier lumen (2) soit en communication fluide avec une source de gonflage.

9. Cathéter selon la revendication 1, comprenant au moins un lumen supplémentaire se prolongeant à travers l'arbre tubulaire (1) et possédant des extrémités proximale et distale ouvertes.

10. Cathéter selon la revendication 9, dans lequel il existe un lumen supplémentaire.

11. Cathéter selon la revendication 10, dans lequel l'extrémité proximale du lumen supplémentaire est reliée à un moyen de communication fluide (32) de façon que l'extrémité distale du lumen soit en communication fluide avec le moyen de communication fluide (32).

12. Système de cathéter à ballon de dilatation comprenant un fil de guidage (8) et deux cathéters (1) ou davantage selon la revendication 1, ces cathéters (1) pouvant être échangés en séquence sur ledit fil de guidage (8).

13. Système selon la revendication 12, dans lequel chaque cathéter (1) possède un ballon de dilatation (4) d'une taille différente.
